# EUROPEAN PATENT SPECIFICATION

(11) **EP 2 575 457 B1**
(45) Date of publication and mention of the grant of the patent: **17.08.2016**
(21) Application number: 10852078.4
(22) Date of filing: 25.05.2010
(51) Int. Cl.: A61K 31/38, A61K 9/50, A61K 9/48

(54) **ORAL PHARMACEUTICAL COMPOSITION OF DULOXETINE**
ORALE PHARMAZEUTISCHE DULOXETINZUSAMMENSETZUNG
COMPOSITION PHARMACEUTIQUE À BASE DE DULOXÉTINE DESTINÉE À LA VOIE ORALE

(43) Date of publication of application: 10.04.2013
(73) Proprietor: Hetero Research Foundation, Hyderabad 500 018, Andhrapradesh (IN)
(72) Inventor: PARTHASARADHI REDDY, Bandi, Hyderabad 500018 (IN); KHADGAPATHI, Podili, Hyderabad 500018 (IN); VENKATA RAMANA REDDY, Sanikommu, Hyderabad 500018 (IN); PRAVEEN KUMAR, Kalidindi, Hyderabad 500018 (IN)
(74) Representative: ZBM Patents ApS
(86) International application number: PCT/IN2010/000353
(87) International publication number: WO 2011/148380

(56) References cited:
- EP-A1- 1 820 800
- EP-A2- 0 693 282
- WO-A1-2011/006670
- US-A1- 2003 096 001
- US-A1- 2006 270 731
- US-A1- 2008 226 711
- US-A1- 2009 175 935
- US-A1- 2009 226 517
- JANSEN P J ET AL: "CHARACTERIZATION OF IMPURITIES FORMED BY INTERACTION OF DULOXETINE HCI WITH ENTERIC POLYMERS HYDROXYPROPYL METHYLCELLULOSE ACETATE SUCCINATE AND HYDROXYPROPYL METHYLCELLULOSE PHTHALATE", JOURNAL OF PHARMACEUTICAL SCIENCES, AMERICAN PHARMACEUTICAL ASSOCIATION, WASHINGTON, US, vol. 87, no. 1, 1 January 1998 (1998-01-01), pages 81-85, XP000891949, ISSN: 0022-3549, DOI: 10.1021/JS970133R

## Description

### Field of the invention

The present invention relates to an oral pharmaceutical composition of duloxetine or pharmaceutically acceptable salts thereof. The invention also relates to a delayed release composition of duloxetine comprising a core containing duloxetine, an optional separating layer, an enteric layer and an optional finishing layer.

### Background of the Invention

Duloxetine is a selective serotonin and norepinephrine reuptake inhibitor (SSNRI), effective for major depressive disorder and it is as effective as venlafaxine in generalized anxiety disorder.

Duloxetine is chemically (+)-(*S*)-*N-*methyl-γ-(1-naphthyloxy)-2-thiophene propylamine, and commonly used as its hydrochloride salt. In this document, the term "duloxetine" will refer to the hydrochloride salt of the S-enantiomer unless otherwise specified. Duloxetine hydrochloride has the following formula.

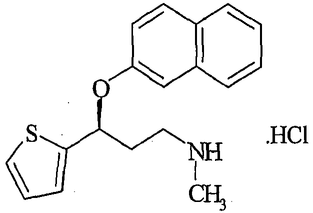

Duloxetine is commercially available as capsules containing delayed release pellets under the trade name CYMBALTA™ in the United States. It has been approved by the FDA for the treatment of major depressive disorder, treatment of generalized anxiety disorder, management of neuropathic pain associated with diabetic peripheral neuropathy and management of fibromyalgia. Duloxetine is also commercially available as hard gastro-resistant capsules under the trade names CYMBALTA™ and YENTREVE™ in Europe. It has been approved by EMEA for the treatment of major depressive disorder, treatment of diabetic peripheral neuropathic pain in adults and treatment of generalized anxiety disorder under the brand name CYMBALTA™ and for the treatment of moderate to severe stress urinary incontinence in woman under the trade name YENTREVE™.

Duloxetine, being an acid-labile substance is very much susceptible to degradation in the acidic environment of the stomach. Therefore duloxetine is formulated as an enteric coated dosage form to protect it from acid degradation.

US Patent No. 5,508,276 discloses an enteric duloxetine pellet comprising hydroxypropylmethyl cellulose acetate succinate (HPMCAS) as an enteric coating polymer. The '276 patent also discloses that the HPMCAS should be neutralized, for example, with ammonia to facilitate its dissolution. The '276 patent also discloses that duloxetine was found to react with many enteric coatings to form a slowly soluble or insoluble coating. This may lead to a disadvantageous drug-releasing profile and/or low bioavailability.

US patent application no. 2006/0165776 describes an oral pharmaceutical composition comprising a core comprising duloxetine or its pharmaceutically acceptable derivative thereof and the said core comprised of pharmaceutically inert nuclei and duloxetine or its pharmaceutically acceptable derivative thereof mixed and compressed together, an intermediate layer and an enteric layer comprising one or more enteric polymers; wherein the said composition is free of alkaline reacting compounds.

US patent application no. 2007/0292511 discloses a duloxetine hydrochloride delayed release formulation, comprising an inert core, a drug layer comprising duloxetine hydrochloride, a separating layer and an enteric layer comprising at least one of methacrylic acid copolymer and hydroxypropyl methyl cellulose phthalate.

US patent application no. 2008/0226711 discloses a delayed release pharmaceutical composition comprising a core comprising an inert core coated with duloxetine, optionally a separating coat on the core and an enteric coat on the core or on the separating coat, wherein the enteric coat comprises hydroxypropyl methylcellulose phthalate (HPMCP) or cellulose acetate phthalate (CAP) or polyvinyl acetate phthalate (PVAP).

Jansen et al, J Pharm Sci, 87 (I), 1998: 81-85 discloses that duloxetine reacts with degradation products or residual free acids present in the enteric polymer such as hydroxypropyl methylcellulose phthalate to form impurities such as phthalamide impurities.

None of the above said prior art discloses the use of carboxymethyl ethyl cellulose as an enteric coating material in the delayed release pharmaceutical composition of duloxetine. The present invention overcomes the above commonly faced stability problems with the enteric coated formulations of duloxetine.

Accordingly, the present invention provides a delayed release composition comprising; inert core, a drug layer comprising duloxetine, an optional separating layer, an enteric layer comprising carboxymethyl ethyl cellulose and an optional finishing layer.

### Objective of the Invention

Accordingly, the main objective of the invention is to provide a delayed release composition of duloxetine or its pharmaceutically acceptable salts comprising; inert core, a drug layer comprising duloxetine, an optional separating layer, an enteric layer comprising carboxy methyl ethyl cellulose and an optional finishing layer.

### Summary of the Invention

Accordingly, the main aspect of the present invention is to provide a delayed release formulation comprising:
a) an inert core loaded with duloxetine or its pharmaceutically acceptable salts.
b) an optional separating layer.
c) an enteric coating over the sub coating with carboxymethyl ethyl cellulose; and
d) an optional finishing layer.

### Detailed description of the Invention

According to the present invention there is provided a delayed release pharmaceutical composition of duloxetine or its pharmaceutically acceptable salts comprising:
a) an inert core loaded with duloxetine or its pharmaceutically acceptable salts.
b) an optional separating layer.
c) an enteric coating over the sub coating with carboxy methyl ethyl cellulose; and
d) an optional finishing layer.

Preferably, duloxetine is in the form of its hydrochloride salt.

Preferably, the inert core comprises sugar spheres or pellets of microcrystalline cellulose and more preferably, the inert core comprises sugar spheres.

The drug layer further comprises one or more pharmaceutically acceptable excipients that do not react adversely with duloxetine.

Preferably, the pharmaceutically acceptable excipients are selected from diluents, binders and disintegrants.

The preferable diluent is selected from the group consisting of mannitol, sucrose, sorbitol, starch, modified starches, xylitol, lactose, microcrystalline cellulose, magnesium carbonate, starch, calcium carbonate, dicalcium phosphate, tribasic calcium phosphate, and calcium sulphate.

The preferable binder is selected from L-hydroxy propyl cellulose, corn starch, polyvinyl pyrrolidine, hydroxyl propyl methyl cellulose, hydroxyl ethyl cellulose and pre-gelatinized starch.

The disintegrant may be preferably selected from croscarmellose sodium, crospovidone, sodium starch glycolate and low substituted hydroxyl propyl cellulose.

More preferably, the pharmaceutically acceptable excipients are selected from sucrose, hydroxy propyl cellulose, hydroxy propyl methyl cellulose, crospovidone, talc and mixtures thereof.

Preferably, the drug layer comprises duloxetine, sugar spheres, hydroxy propyl methyl cellulose, hydroxy propyl cellulose, sucrose extra fine, crospovidone and talc.

More preferably, the drug layer comprises about 15% to about 40% duloxetine, about 30-80% sugar spheres, about 2-10% hydroxy propyl methyl cellulose, about 0-10% hydroxy propyl cellulose, about 0-10% sucrose extra fine, about 1-10% crospovidone and about 1-10% talc, wherein the percentages are by weight of the drug layer.

The separating layer between drug layer and enteric layer is optional. The functions of the separating layer, if applied, are to provide a smooth base for the application of the enteric layer, to prolong the pellet's resistance to acid conditions and to improve stability.

The preferable separating layer comprises sucrose, hydroxy propyl methyl cellulose and talc.

Preferably, the separating layer is present in an amount of about 5-30% based on the total weight of the formulation.

More preferably, the separating layer is present in an amount of about 10-25% based on the total weight of the formulation.

The enteric layer may preferably comprise carboxymethyl ethyl cellulose and povidone.

The enteric layer is present in an amount of about 5-30% based on the total weight of the formulation.

More preferably, the enteric layer is present in an amount of about 10-20% based on the total weight of the formulation.

The solvent used to make the enteric coating solution is selected from isopropanol, water and mixtures thereof. The preferable solvent is a mixture of isopropanol and water.

The preferable ratio of isopropanol and water used in the enteric coating solution is 1:9 to 9:1.

Optionally, a finishing layer is present over the enteric coating layer.

Preferably, the optional finishing layer comprises hydroxypropyl methyl cellulose, talc, polyethylene glycol 400 (PEG-400) and titanium dioxide.

Preferably, the delayed release pharmaceutical composition of duloxetine is in the form of pellets.

The film coated duloxetine pellets were then filled into hard gelatin capsules.

The following examples further exemplify the invention and are not intended to limit the scope of the invention.

**Example 1:**

| **Ingredient** | **Quantity** (**mg**) |
|---|---|
| **Core** | |
| Sugar spheres | 155.70 |
| Duloxetine hydrochloride | 67.30 |
| Hydroxypropyl methyl cellulose | 10.00 |
| Hydroxy propyl cellulose | 3.00 |
| Sucrose extra fine | 3.00 |
| Crospovidone | 5.00 |
| Talc | 6.00 |
| Purified water | q.s |

| **Separating layer** | |
|---|---|
| Sucrose | 31.00 |
| Hydroxypropyl methyl cellulose | 11.00 |
| Talc | 11.00 |
| Purified water | q.s |

| **Enteric Coating** | |
|---|---|
| Carboxy methyl ethyl cellulose | 39.00 |
| Povidone | 3.00 |
| Isopropanol /Water (7:3) | q.s |
| **Total weight** | **345.00** |

**Example 2:**

| **Ingredient** | **Quantity** (**mg**) |
|---|---|
| **Core** | |
| Sugar spheres | 155.70 |
| Duloxetine hydrochloride | 67.30 |
| Hydroxypropyl methyl cellulose | 10.00 |
| Sucrose extra fine | 3.00 |
| Crospovidone | 5.00 |
| Talc | 6.00 |
| Purified water | q.s |

| **Separating layer** | |
|---|---|
| Sucrose | 31.00 |
| Hydroxypropyl methyl cellulose | 11.00 |
| Talc | 11.00 |
| Purified water | q.s |

| **Enteric Coating** | |
|---|---|
| Carboxy methyl ethyl cellulose | 39.00 |
| Povidone | 3.00 |
| Isopropanol/Water (8:2) | q.s |
| **Total weight** | **342.00** |

**Example 3:**

| **Ingredient** | **Quantity (mg)** |
|---|---|
| **Core** | |
| Sugar spheres | 155.70 |
| Duloxetine hydrochloride | 67.30 |
| Hydroxypropyl methyl cellulose | 10.00 |
| Sucrose extra fine | 3.00 |
| Crospovidone | 5.00 |
| Talc | 6.00 |
| Purified water | q.s |

| **Separating layer** | |
|---|---|
| Sucrose | 22.00 |
| Hydroxypropyl methyl cellulose | 5.00 |
| Talc | 10.00 |
| Purified water | q.s |

| **Enteric Coating** | |
|---|---|
| Carboxy methyl ethyl cellulose | 41.00 |
| Povidone | 3.10 |
| Isopropanol /Water (7:3) | q.s |
| **Total weight** | **328.10** |

**Example 4:**

| **Ingredient** | **Quantity (mg)** |
|---|---|
| **Core** | |
| Sugar spheres | 155.70 |
| Duloxetine hydrochloride | 67.30 |
| Hydroxypropyl methyl cellulose | 13.00 |
| Hydroxypropyl cellulose | 3.00 |
| Crospovidone | 5.00 |
| Talc | 6.00 |
| Purified water | q.s |

| **Separating layer** | |
|---|---|
| Sucrose | 42.40 |
| Hydroxypropyl methyl cellulose | 6.60 |
| Talc | 13.00 |
| Purified water | q.s |

| **Enteric Coating** | |
|---|---|
| Carboxy methyl ethyl cellulose | 41.80 |
| Povidone | 3.20 |
| Isopropanol/Water (7:3) | q.s |
| **Total weight** | **357.00** |

**Example 5:**

| **Ingredient** | **Quantity (mg)** |
|---|---|
| **Core** | |
| Sugar spheres | 155.70 |
| Duloxetine hydrochloride | 67.30 |
| Hydroxypropyl methyl cellulose | 10.00 |
| Sucrose extra fine | 3.00 |
| Crospovidone | 5.00 |
| Talc | 6.00 |
| Purified water | q.s |

| **Separating layer** | |
|---|---|
| Sucrose | 32.00 |
| Hydroxypropyl methyl cellulose | 5.00 |
| Talc | 10.00 |
| Purified water | q.s |

| **Enteric Coating** | |
|---|---|
| Carboxy methyl ethyl cellulose | 41.00 |
| Povidone | 3.10 |
| Isopropanol/Water (7:3) | q.s |
| **Total weight** | **338.10** |

### Preparation process:

The process for preparation of formulations of examples 1-5 as given below:

### Core

Duloxetine hydrochloride and other inactive ingredients were dispersed in purified water. This dispersion was coated on the sugar spheres in a fluid bed processor.

### Separating Layer

Sucrose and hydroxypropyl methyl cellulose were dissolved in purified water and talc dispersed in to this solution under stirring. This dispersion was coated on the drug loaded spheres in a fluid bed processor.

### Enteric Layer

In order to provide enteric coated duloxetine pellets, the above coated particles were then coated in fluid bed processor with a solution of carboxymethyl cellulose, povidone in purified water and isopropanol.

### Finishing layer

Hydroxypropyl methyl cellulose, polyethylene glycol, titanium dioxide and talc were added in purified water and this dispersion was coated on the enteric coated pellets in a fluid bed processor.

### Capsule filling

The above coated pellets were then filled into hard gelatin capsules.

## Claims

1. A delayed release pharmaceutical composition of duloxetine or its pharmaceutically acceptable salts comprising:
a) an inert core loaded with duloxetine or its pharmaceutically acceptable salts.
b) an optional separating layer.
c) an enteric coating over the sub coating with carboxymethyl ethyl cellulose and povidone; and
d) an optional finishing layer.

2. The delayed release pharmaceutical composition of claim 1, wherein the duloxetine is in the form of its hydrochloride salt.

3. The delayed release pharmaceutical composition of claim 1, wherein the inert core comprises sugar spheres or pellets of microcrystalline cellulose.

4. The delayed release pharmaceutical composition of claim 1, wherein the drug layer comprises one or more pharmaceutically acceptable excipients that do not react adversely with duloxetine selected from diluents, binders and disintegrants.

5. The delayed release pharmaceutical composition of claim 4, wherein the pharmaceutically acceptable binder is selected from hydroxypropyl cellulose, corn starch, polyvinyl pyrrolidine, hydroxyl propyl methyl cellulose, hydroxyethyl cellulose and pre-gelatinized starch.

6. The delayed release pharmaceutical composition of claim 4, wherein the disintegrant is selected from croscarmellose sodium, crospovidone, sodium starch glycolate and low substituted hydroxyl propyl cellulose.

7. The delayed release pharmaceutical composition of claim 1, wherein the drug layer comprises 15% to about 40% duloxetine hydrochloride, about 30-80% sugar spheres, about 2-10% hydroxypropyl methyl cellulose, about 0-10% hydroxypropyl cellulose, about 0-10% sucrose extra fine, about 1-10% crospovidone and about 1-10% talc based on the weight of the drug layer.

8. The delayed release pharmaceutical composition of claim 1, wherein the separating layer comprises sucrose, hydroxypropyl methyl cellulose and talc.

9. The delayed release pharmaceutical composition of claim 1, wherein the separating layer is present in an amount of about 5-30% based on the total weight of the formulation.

10. The delayed release pharmaceutical composition of claim 1, wherein the separating layer is present in an amount of about 10-25% based on the total weight of the formulation.

11. The delayed release pharmaceutical composition of claim 1, wherein the enteric layer is present in an amount of about 5-30% based on the total weight of the formulation.

12. The delayed release pharmaceutical composition of claim 1, wherein the composition comprises a finishing layer over the enteric coating.

13. The delayed release pharmaceutical composition of claim 12, wherein the finishing layer comprises hydroxypropyl methyl cellulose, polyethylene glycol, titanium dioxide and talc.

14. The delayed release pharmaceutical composition of claim 1, wherein the delayed release pharmaceutical composition of duloxetine is in the form of pellets.

## Patentansprüche

1. Eine pharmazeutische Zusammensetzung verzögerter Freisetzung aus Duloxetin oder dessen pharmazeutisch akzeptablen Salzen umfassend:
a) einen mit Duloxetin oder dessen pharmazeutisch akzeptablen Salzen geladenen inerten Kern,
b) eine fakultative Trennschicht,
c) eine magensaftresistente Beschichtung oberhalb der unteren Beschichtung mit Carboxymethylethylcellulose und Povidon; und
b) eine fakultative Deckschicht.

2. Die pharmazeutische Zusammensetzung verzögerter Freisetzung des Anspruchs 1, wobei das Duloxetin in Form dessen Hydrochloridsalz vorhanden ist.

3. Die pharmazeutische Zusammensetzung verzögerter Freisetzung des Anspruchs 1, wobei der inerte Kern Zuckerkugeln oder Pellets aus mikrokristalliner Cellulose umfasst.

4. Die pharmazeutische Zusammensetzung verzögerter Freisetzung des Anspruchs 1, wobei die Arzneimittelschicht einen oder mehrere pharmazeutisch akzeptable Hilfsstoffe umfasst, die nicht mit Duloxetin nachteilig reagieren, welche aus Verdünnern, Bindemitteln und Desintegranten ausgewählt sind.

5. Die pharmazeutische Zusammensetzung verzögerter Freisetzung des Anspruchs 4, wobei das pharmazeutisch akzeptable Bindemittel aus Hydroxypropilcellulose, Maisstärke, Polyvinylpyrrolidon, Hydroxylpropylmethylcellulose, Hydroxyethylcellulose und vorgelierter Stärke ausgewählt ist.

6. Die pharmazeutische Zusammensetzung verzögerter Freisetzung des Anspruchs 4, wobei der Desintegrant aus Croscarmellose-Natrium, Crospovidon, Natriumstärkeglycolat und niedrig substituierter Hydroxylpropylcellulose ausgewählt ist.

7. Die pharmazeutische Zusammensetzung verzögerter Freisetzung des Anspruchs 1, wobei die Arzneimittelschicht von 15% bis ca. 40% Duloxetinhydrochlorid, ca. 30-80% Zuckerkugeln, ca. 2-10% Hydroxypropylmethylcellulose, ca. 0-10% Hydroxypropylcellulose, ca. 0-10% extrafeine Saccharose, ca. 1-10% Crospovidon und ca. 1-10% Talk bezogen auf das Gewicht der Arzneimittelschicht umfasst.

8. Die pharmazeutische Zusammensetzung verzögerter Freisetzung des Anspruchs 1, wobei die Trennschicht Saccharose, Hydroxypropylmethylcellulose und Talk umfasst.

9. Die pharmazeutische Zusammensetzung verzögerter Freisetzung des Anspruchs 1, wobei die Trennschicht in einer Menge von ca. 5-30% bezogen auf das gesamte Gewicht der Rezeptur vorhanden ist.

10. Die pharmazeutische Zusammensetzung verzögerter Freisetzung des Anspruchs 1, wobei die Trennschicht in einer Menge von ca. 10-25% bezogen auf das gesamte Gewicht der Rezeptur vorhanden ist.

11. Die pharmazeutische Zusammensetzung verzögerter Freisetzung des Anspruchs 1, wobei die magensaftresistente Schicht in einer Menge von ca. 5-30% bezogen auf das gesamte Gewicht der Rezeptur vorhanden ist.

12. Die pharmazeutische Zusammensetzung verzögerter Freisetzung des Anspruchs 1, wobei die Zusammensetzung eine Deckschicht oberhalb der magensaftresistenten Beschichtung umfasst.

13. Die pharmazeutische Zusammensetzung verzögerter Freisetzung des Anspruchs 12, wobei die Deckschicht Hydroxypropylmethylcellulose, Polyethylenglycol, Titaniumdioxid und Talk umfasst.

14. Die pharmazeutische Zusammensetzung verzögerter Freisetzung des Anspruchs 1, wobei die pharmazeutische Zusammensetzung verzögerter Freisetzung aus Duloxetin in Form von Pellets vorhanden ist.

## Revendications

1. Une composition pharmaceutique à libération retardée de duloxétine ou ses sels pharmaceutiquement acceptables comprenant :
a) un noyau inerte chargé avec de la duloxétine ou ses sels pharmaceutiquement acceptables,
b) une couche de séparation optionnelle,
c) un enrobage entérique au-dessus du sous-enrobage avec carboxyméthyléthylcellulose et povidone ; et
d) une couche de finition facultative.

2. La composition pharmaceutique à libération retardée de la revendication 1, dans laquelle la duloxétine est sous forme de son sel chlorhydrate.

3. La composition pharmaceutique à libération retardée de la revendication 1, dans laquelle le noyau inerte comprend des sphères de sucre ou des pellets de cellulose microcrystalline.

4. La composition pharmaceutique à libération retardée de la revendication 1, dans laquelle la couche de médicament comprend un ou plusieurs excipients pharmaceutiquement acceptables qui ne réagissent pas de façon défavorable avec la duloxétine choisis parmi des diluants, des agglutinants et des agents désintégrants.

5. La composition pharmaceutique à libération retardée de la revendication 4, dans laquelle l'agglutinant pharmaceutiquement acceptable est choisi parmi la hydroxypropylcellulose, l'amidon de maïs, la polyvinylpyrrolidone, l'hydroxylpropylméthylcellulose, l'hydroxyéthylcellulose et l'amidon prégélatinisé.

6. La composition pharmaceutique à libération retardée de la revendication 4, dans laquelle l'agent désintégrant est choisi parmi la croscamellose de sodium, la crospovidone, le glycolate d'amidon de sodium et l'hydroxypropylcellulose faiblement substituée.

7. La composition pharmaceutique à libération retardée de la revendication 1, dans laquelle la couche de médicament comprend de 15% à environ 40% de chlorhydrate de duloxétine, environ 30-80% de sphères de sucre, environ 2-10% d'hydroxypropylméthylcellulose, environ 0-10% d'hydroxypropylcellulose, environ 0-10% de saccharose extra fin, environ 1-10% de crospovidone et environ 1-10% de talc basé sur le poids de la couche de médicament.

8. La composition pharmaceutique à libération retardée de la revendication 1, dans laquelle la couche de séparation comprend du saccharose, de l'hydroxypropylméthylcellulose et du talc.

9. La composition pharmaceutique à libération retardée de la revendication 1, dans laquelle la couche de séparation est présente dans une quantité d'environ 5-30% basé sur le poids total de la formulation.

10. La composition pharmaceutique à libération retardée de la revendication 1, dans laquelle la couche de séparation est présente dans une quantité d'environ 10-25% basé sur le poids total de la formulation.

11. La composition pharmaceutique à libération retardée de la revendication 1, dans laquelle la couche entérique est présente dans une quantité d'environ 5-30% basé sur le poids total de la formulation.

12. La composition pharmaceutique à libération retardée de la revendication 1, dans laquelle la composition comprend une couche de finition au-dessous de l'enrobage entérique.

13. La composition pharmaceutique à libération retardée de la revendication 12, dans laquelle la couche de finition comprend de l'hydroxypropylméthylcellulose, du polyéthylène glycol, de l'oxyde de titane et du talc.

14. La composition pharmaceutique à libération retardée de la revendication 1, dans laquelle la composition pharmaceutique à libération retardée de duloxétine est sous forme de pellets.
